# EUROPÄISCHE PATENTSCHRIFT

(11) **EP 2 034 947 B1**
(45) Veröffentlichungstag und Bekanntmachung des Hinweises auf die Patenterteilung: **07.12.2011**
(21) Anmeldenummer: 07726065.1
(22) Anmeldetag: 18.06.2007
(51) Int. Cl.: A61K 6/06

(54) **MATERIAL UND ROHLING FÜR ZAHNERSATZ**
MATERIAL AND BLANK FOR DENTURES
MATÉRIAU ET ÉBAUCHE POUR PROTHÈSES DENTAIRES

(30) Priorität: 23.06.2006 EP 06012980
(43) Veröffentlichungstag der Anmeldung: 18.03.2009
(73) Patentinhaber: Institut Straumann AG, 4002 Basel (CH)
(72) Erfinder: HOLZNER, Stephan, 80269 Hohenschäftlarn (DE); WEBER, Gerhard, 86932 Pürgen (DE)
(74) Vertreter: Grünecker, Kinkeldey, Stockmair & Schwanhäusser
(86) Internationale Anmeldenummer: PCT/EP2007/005360
(87) Internationale Veröffentlichungsnummer: WO 2007/147549

(56) Entgegenhaltungen:
- EP-A1- 0 140 638
- EP-A1- 0 218 853
- EP-A1- 0 624 360
- EP-A2- 0 630 622
- WO-A-01/12132
- WO-A-97/30654
- WO-A-02/074714
- WO-A-02/085242
- US-B1- 6 409 852
- DATABASE WPI Week 200446 Derwent Publications Ltd., London, GB; AN 2004-481264 XP002408114 & CN 1 489 988 A (SHANGHAI SILICATE INST CHINESE ACAD SCI) 21. April 2004 (2004-04-21)
- KOSMAC T ET AL: "STRENGTH AND RELIABILITY OF SURFACE TREATED Y-TZP DENTAL CERAMICS" JOURNAL OF BIOMEDICAL MATERIALS RESEARCH, WILEY, NEW YORK, NY, US, Bd. 53, Nr. 4, 2000, Seiten 304-313, XP000972222 ISSN: 0021-9304

## Beschreibung

Die Erfindung betrifft die Herstellung von Zahnersatzteilen.

Zahnersatzteile können mittels automatisierter Herstellungsmethoden aus Rohmaterialien wie Keramiken oder Metallen gefertigt werden. Hierbei werden die Zahnersatzteile z. B. aus Vollmaterialien gefräst. Im Falle von keramischen Materialien werden die gefrästen Zahnersatzteile in der Regel dicht gesintert, um so Zahnersatzteile mit einer hohen Bruchfestigkeit zu erhalten.

WO 02/074714 A1 offenbart die Verwendung eines auf Zirkonoxid basierenden Pulvers, das gepresst und dann gesintert werden kann, wodurch sich eine mechanische Festigkeit von 1200 MPa ergibt. Eine weitere Behandlung bei einer Temperatur von 1450°C und unter einem Druck von 2000 bar kann je nach Sintertemperatur (1450°C bzw. 1600°C) eine mechanische Festigkeit zwischen von 1700 MPa oder 1850 MPa ergeben.

EP 0 218 853 A1 offenbart verschiedene Verfahren zur Herstellung von gesintertem Zirkonoxidmaterial, wobei ein Rohling durch Pressen von Pulver hergstellt und dieser Rohling mittels isostatischem Heißpressen (HIP) gesintert werden kann. Durch das HIP bei Temperaturen von 1150°C bis 1500°C unter einem Druck zwischen 1000 kg/cm² und 2200 kg/cm² kann eine mechanische Festigkeit von bis zu 1700MPa erreicht werden.

EP 0 140 638 A1 offenbart einen gesinterten Zirkonoxidkörper, der je nach Gewichtsanteilen der einzelnen Bestandteile, Drei-Punkt-Biegefestigkeiten bis zu 2510 MPa aufweisen kann, wobei ein Heißpressen unter einen Druck von mindestens 50 MPa bei einer Temperatur zwischen 1300°C und 1700°C vorgenommen wurde.

EP 0 624 360 A1 offenbart die Verwendung von Yttriumoxid-stabilisiertem Zirkonoxid zur Herstellung von Prothesen, die aus einem dichtgesinterten Halbzeug durch maschinelle Bearbeitung hergestellt werden können.

EP 0 630 622 A2 offenbart ein Verfahren zur Herstellung von keramischen Zahnprothesen, wobei ein dichtgesintertes Halbzeug verwendet werden kann. Ein poröser Rohling kann zu einer Prothese umgearbeitet werden, wobei die poröse Prothese mit Masszugaben als Zwischenprodukt dichtgesintert und anschließend auf die Endform und -masse bearbeitet werden kann.

WO 01/12132 A1 offenbart die Verwendung von Keramikrohlingen auf Zirkonoxidbasis zur Herstellung von Zahnersatz. Nach der Bearbeitung der Rohlinge, die beispielsweise durch isostatisches Pressen von Granulat und Entbindern der Presslinge hergestellt werden, werden diese bei 1200°C bis 1650°C dichtgesintert, wobei sich eine Bruchfestigkeit von mehr als 1000 MPa ergeben kann.

Database WPI week 200446 Derwent Publications Ltd. (AN 2004 - 481264 and PN CN 1 489 988 A) offenbart eine dichtgesinterte Yttriumoxid-stabilisierte Zirkonoxidkeramik für die Zahnreparatur mit einer hohen Biege- und Bruchfestigkeit, wobei die Keramik durch ein Sintern von Pulver bei 1250°C hergestellt wird.

WO 02/064099 A1 offenbart ein Verfahren zur Herstellung von Zahnersatz, wobei vorgesinterte Rohlinge mit einer Rohbruchfestigkeit von 31 bis 50 MPa verwendet werden, die durch ein fräsendes Verfahren bearbeitet und anschließend in einem Temperaturbereich von 1200 bis 1650°C dichtgesintert werden. Es kann eine Festigkeit von mehr als 1000 MPa erreicht werden.

Aufgabe der vorliegenden Erfindung ist es, die Ausgangsstoffe von Zahnersatzteilen, Rohlinge und dazugehörige Verfahren und damit auch die Zahnersatzteile zu verbessern.

Diese Aufgabe wird gelöst durch, ein Verfahren zum Herstellen von einem Zahnersatzteil oder von Zahnersatzteilen nach Anspruch 1.

Bevorzugte Ausführungsformen sind in den abhängigen Ansprüchen offenbart.

Durch aufwendige Untersuchungen hat sich herausgestellt, dass es möglich ist, Material für einen Zahnersatz zu schaffen, das eine 3-Punkt-Biegefestigkeit von mindestens 1300 MPa hat. Das Material ist bevorzugterweise ein keramisches Material wie etwa Zirkonoxid oder umfasst Zirkonoxid. Statt Zirkonoxid wird für das selbe Material oft auch der Begriff Zirkoniumdioxid verwendet.

Sämtliche Angaben über eine 3-Punkt-Biegefestigkeit in diesem Dokument beziehen sich auf die in der Norm ISO 6872 definierte 3-Punkt-Biegefestigkeit.

Vorteilhaft ist weiterhin ein Material, das eine 3-Punkt-Biegefestigkeit von mindestens 1400, 1500, 1600, 1700, 1800, 1900 oder 2000 MPa aufweist. Je höher die 3-Punkt-Biegefestigkeit ist, desto höher ist auch die Belastbarkeit von Zahnersatzteilen, die aus diesem Material hergestellt wurden, so dass Zahnersatzteile mit dünneren Wandstärken oder solche, die größeren Belastungen standhalten, möglich werden.

Weiterhin ist es vorteilhaft, wenn das Material Dichtschwankungen von weniger als 10%, 5% oder 2% aufweist. Derartige Dichtschwankungen führen zu einer inhomogenen Druckfestigkeit, so dass es vorkommen kann, dass ungünstigerweise genau an einer hoch belasteten Stelle des Zahnersatzteils das Material nur eine geringe Dichte und somit eine geringe Bruchfestigkeit aufweist.

Das Material kann weiterhin einen hohen Zirkonoxid sowie zumindest einen gewissen Yttriumoxidanteil aufweisen. Weiter kann Hafniumoxid hinzugesetzt werden und/oder ein Metalloxid oder Metallsalz.

Das Material kann beispielsweise einen Zirkonoxidanteil über 90, 92, 94, 96 oder 97 % aufweisen, jedoch nicht mehr als 92, 94, 96, 98 oder 99 %. Hafniumoxid kann gar nicht oder mit mindestens 0,5, 1, 1,5, 2, 2,5, 3 % und/oder mit nicht mehr als 0,5, 1, 1,5, 2, 2,5, 3, 3,5, 4 % vorliegen. Yttriumoxid liegt mit einem Anteil von mehr als 1,5, 2, 2,5, 3, 3,5, 4, 4,5, 5 % und/oder mit einem Anteil von nicht höher als 2, 2,5, 3, 3,5, 4, 4,5, 5, 5,5, 6 % vor.

Weiterhin kann mit einem Anteil von mindestens 0,01, 0,02, 0,05, 0,1, 0,2, 0,3, 0,4, 0,5 % und/oder mit einem Anteil von nicht mehr als 0,1, 0,2, 0,3, 0,4, 0,5, 0,6, 0,7, 0,75 % eines oder mehrere der Oxide von Aluminium, Gallium, Germanium, Indium, Zinn, Blei, Lanthaniten, Metallen, Eisen und/oder einem oder mehreren Metallsalzen vorliegen.

Die in diesem Dokument verwendeten Prozentangaben der chemischen Zusammensetzung beziehen sich auf Gewichtsprozent.

Die letzteren Oxide oder Salze sind bei der Herstellung des Materials möglichst homogen verteilt worden, so dass sich Kristallisationskeime und damit auch Kristallitgrößen möglichst homogen ergeben. Weiterhin können hierdurch die Kristallisationsachsenausrichtungen beeinflusst werden und zwar dahingehend, dass sie möglichst gleichmäßig in alle Raumrichtungen verteilt sind, so dass sich beim Dichtsintern, wodurch dann das Zahnersatzteil erzeugt wird, eine Kontraktion des Materials in allen Raumrichtungen der gleichen Größe ergibt.

Das Dichtsintern erfolgt z. B. bei Temperaturen zwischen 1200 °C und 1600 °C, wie etwa bei 1400 °C über einen Zeitraum von 10 bis 16 Stunden. Bevorzugt ist ein Dichtsintern bei 1400 °C für 14 Stunden. Das Dichtsintern erfolgt weiterhin vorzugsweise bei Umgebungsdruck, da dies mit relativ einfachen Öfen möglich ist. Es kann jedoch auch ein erhöhter Druck beim Sintern (bis 200 bar) anliegen. Dies heißt z. B. dass der maximale Pressdruck bei Raumtemperatur oder einer Temperatur von weniger als 50 °C, 100 °C oder 200 °C angelegt ist. Auch kann der gesamte Pressvorgang bei Raumtemperatur oder einer Temperatur von weniger als 50 °C, 100 °C oder 200 °C durchgeführt werden.

Das Zahnersatzteil besteht aus einem der oben oder weiter unten genannten Materialien.

Das Material kann durch Dichtsintern in ein Material für Zahnersatz umgewandelt werden. Dieses Rohlingsmaterial hat eine Biegefestigkeit von mindestens 20 und höchstens 180 MPa und sämtliche Zwischenwerte oder Zwischenintervalle sind möglich. Je geringer diese 3-Punkt-Biegefestigkeit ist, desto leichter lässt sich das Material beispielsweise durch Fräsen und dabei ohne hohen Fräswerkzeugverschleiß bearbeiten. Je höher die Biegefestigkeit ist, desto höhere Belastungen hält das Material beim Fräsen aus, so dass ein schnelleres Fräsen möglich wird. In dem genannten Intervall ist ein guter Kompromiss zwischen den verschiedenen Anforderungen gegeben.

Die chemische Zusammensetzung dieses Rohlings-Materials entspricht der Zusammensetzung des Materials für Zahnersatz, da sich beim Dichtsintern die chemische Komposition nicht ändert.

Ein Rohling zum Herstellen von Zahnersatz weist vorzugsweise ein oben genanntes Material für einen Rohling zum Herstellen von Zahnersatz auf.

Weiterhin ist ein Rohling zur Herstellung von Zahnersatz dadurch gekennzeichnet, dass das Material des Rohlings bei einem Pressdruck von mindestens 1500 bar isostatisch oder uniaxial oder biaxial oder triaxial gepresst wurde.

Es hat hierbei eine Dichte von 3,10 - 3,30 g/m³, vorzugsweise 3,15 - 3,25 g/cm³.

Es hat sich gezeigt, dass durch das Pressen mit einem sehr hohen Pressdruck das Rohlingsmaterial nach dem Dichtsintern eine sehr hohe 3-Punkt-Biegefestigkeit aufweist. Auch wenn sich die Dichte nach dem Dichtsintern praktisch unabhängig von dem Rohlingspressdruck zeigt, so ergibt sich überraschenderweise hier aber doch eine Verbesserung der 3-Punkt-Biegefestigkeit aufgrund eines hohen Pressdrucks. Beim gesamten Pressvorgang bzw. beim Maximaldruck beträgt die Temperatur Raumtemperatur oder weniger als 50 °C, 100 °C oder 200 °C.

Die Dichte des dichtgesinterten Materials beträgt vorzugsweise 6,00 bis 6,10 oder 6,05 bis 6,07 g/cm³.

Insbesondere hat sich hierbei gezeigt, dass sich die 3-Punkt-Biegefestigkeit von einem grö-βeren Rohlingskörper, aus dem mehrere Rohlinge gewonnen werden können, sehr zuverlässig für sämtliche Rohlinge des Rohlingskörpers ergibt. Die Variation der 3-Punkt-Biegefestigkeit innerhalb eines derart hergestellten Rohlingskörpers ist relativ gering.

Der Pressdruck kann auch höher als 1500 bar sein, d. h. bis zu 3000 oder 4000 bar. Das Pressen des Materials zum Herstellen des Rohlings kann bei Umgebungstemperatur oder auch erhöhter Temperatur, z. B. 50 °C, 100 °C oder 200 °C, stattfinden.

Der Rohling hat vorzugsweise eine Scheibenform, wobei sich hierbei eine kreisrunde, quadratische, rechteckige oder polygonale Scheibenform als vorteilhaft herausgestellt hat, für die weitere Bearbeitung zur Gewinnung von Zahnersatzteilen aus dem Rohling.

An der Scheibenseite kann eine Markierung entweder durch eine Nut oder eine farbige oder sonstartige Markierung vorgesehen sein, mit der die Orientierung des Rohlings eindeutig festlegbar ist.

Weiterhin ist es vorteilhaft, wenn die Scheibenseite keine Stufen aufweist, da sich dann eine einfache geometrische Form des Rohlings ergibt. Beim Herstellen von Zahnersatzteilen müssen die Zahnersatzteile in einer vergrößerten Form aus dem Rohling herausgearbeitet werden, da sich beim nachfolgenden Dichtsintern das Material kontrahiert. Der Vergrößerungsfaktor entspricht hierbei der dritten Wurzel aus dem Quotient der Zieldichte und der Vordichtsinterungsdichte. Dadurch, dass die Scheibenseite keine Stufen aufweist, lässt sich das Volumen der Scheibe möglichst exakt und einfach durch Abmessen der Größe des Rohlings bestimmen, so dass auch die Vorsinterungsdichte möglichst exakt und leicht bestimmt werden kann.

Wie bereits oben erwähnt, führt das Herstellen eines Rohlings zum Herstellen von Zahnersatz durch Pressen mit einem hohen Pressdruck zu einer guten Homogenität des Rohlingsmaterials. Deshalb ist im weiteren ein Satz von Rohlingen, insbesondere von Rohlingen aus ein und demselben Rohlingskörper gegeben, wobei die Rohlinge mittels Dichtsintern eine 3-Punkt-Biegefestigkeit von mindestens 1300 bis 2000 MPa aufweisen können.

Weiterhin ist ein Satz von Rohlingen aus ein und demselben Rohlingskörper dadurch gekennzeichnet, dass der Rohlingskörper bei einem Pressdruck von mindestens 1500 bar isostatisch, uniaxial, biaxial oder triaxial gepresst wurde.

Auch betrifft die Erfindung ein Verfahren zum Herstellen von Rohlingskörpern oder Rohlingen für die Herstellung von Zahnersatz. Bei den Verfahren wird ein Pulver mit einem isostatischen oder uniaxialen, biaxialen oder triaxialen Pressdruck von mindestens 1500 bar zu einem Rohlingskörper gepresst. Der Druck kann auch 4100 bar oder einen Zwischenwert zwischen 1500 und 4100 bar betragen oder in sämtlichen Zwischenintervallen zwischen 1500 und 4100 bar liegen.

Ein derartig hergestellter Rohlingskörper oder Rohling kann eine 3-Punkt-Biegefestigkeit im Bereich von zwischen 20 und 180 MPa oder sämtlichen Zwischenwerten oder aus möglichen Zwischenintervallen zwischen diesen Werten aufweisen. Damit ist eine Bearbeitbarkeit des Rohlingsmaterials gegeben. Wird dieses Material dichtgesintert, so ergibt sich eine 3-Punkt-Biegefestigkeit von mindestens 1300 bis zu 2000 MPa. Bei dem Verfahren wird das Pulver zur Pressung beispielsweise in einem gummi-elastischen Körper eingefüllt. Das Befüllen findet vorzugsweise in einem Sauberraum statt, so dass ungewünschte Verunreinigungen des Pulvers möglichst klein gehalten werden.

Das Pulver kann ein Oxid mit evtl. einer Metallsalzbeimischung sein, wie sie weiter oben angegeben worden ist.

Das Pressen findet vorzugsweise in einer hydrostatischen Presse statt, mit der ein isostatisches Pressen des Pulvers möglich wird. Ein derartig gepresstes Pulver führt zu einem Rohling mit einer sehr homogenen Dichte einer guten Materialkontraktion beim Dichtsintern in der Hinsicht, dass die Kontraktion in verschiedenen Raumrichtungen gleich groß ist. Durch das Pressen ergibt sich ein Rohlingskörper, der in der Regel nicht völlig regelmäßig geformt ist. Insbesondere beim isostatischen Pressen ergibt sich ein Rohlingskörper mit einer gewellten Oberfläche. Es ist daher vorteilhaft, einen solchen Rohlingskörper nach dem Pressen zu überdrehen oder plan zu bearbeiten. Das Überdrehen führt zu einer kreiszylindrischen Form. Beim Planbearbeiten werden Oberflächen des Rohlingskörpers so bearbeitet, dass sie anschließend eben oder plan sind. Dies führt beispielsweise zu Rohlingskörpern mit einem quadratischen oder rechteckigen Querschnitt, wenn die verschiedenen hergestellten Ebenen senkrecht zueinander sind oder zu einem sonstigen polygonalen Querschnitt.

Bei dem Verfahren wird der Rohlingskörper vorzugsweise in mehrere Rohlinge zerlegt. Dies geschieht vorzugsweise durch Trennen entlang von Ebenen, die vorzugsweise parallel zueinander und in etwa senkrecht zu einer Längsachse des Rohlingskörpers liegen, mit dem Verfahren ist es jedoch auch möglich, unmittelbar Rohlinge herzustellen und nicht erst Rohlingskörper, die dann zertrennt werden. So können beispielsweise auch bei einem einzelnen Pressvorgang mehrere Rohlinge separat hergestellt werden. Auch können bei einem einzelnen Pressvorgang mehrere Rohlingskörper separat hergestellt werden, jeder in einem separaten gummi-elastischen Körper.

Der Rohling kann an der scheibenseite eine Markierung kann wie etwa eine Nut und oder eine farbige Linie und/oder einen Bar-Code aufweisen. Die Erfindung betrifft auch ein Verfahren zum Herstellen eines Zahnersatzteils unter Verwendung eines der beschriebenen Rohlinge oder eines nach dem beschriebenen Verfahren hergestellten Rohlings. Die Bearbeitung ist vorzugsweise Fräsen.

Bei irgendeinem der obigen Verfahren oder Rohlinge oder Materialien kann während des gesamten Pressvorgangs oder beim Maximaldruck Umgebungstemperatur oder eine Temperatur von weniger als 50 °C, 100 °C oder 200 °C, vorhanden sein.

Beim gesamten Sintern oder bei der Maximaltemperatur liegt bevorzugterweise Umgebungsdruck vor. Es kann jedoch auch ein Überdruck von nicht mehr als 1, 2, 5, 10, 20, 50, 100 oder 200 bar vorliegen.

Beim Sintern ist das Sintergut, z. B. ein Material, Rohling oder Zahnersatzteil, vorzugsweise nicht eingepackt. Es kann vielmehr lose in einem Ofen liegen. Insbesondere ist keine flüssigkeitsdichte Verpackung vorgesehen.

Vorteilhafte Ausführungsformen der Erfindung sollen anhand von den beiden Figuren erläutert werden. Dabei zeigt:
- Fig. 1: eine schematische Schnittansicht einer isostatischen Presse,
- Fig. 2: dreidimensionale schematische Ansichten von Rohlingskörpem,
- Fig. 3: eine dreidimensionale schematische Ansicht von einem Rohlingskörper und daraus gewonnenen Rohlingen,
- Fig. 4: eine dreidimensionale schematische Ansicht eines Rohlings.

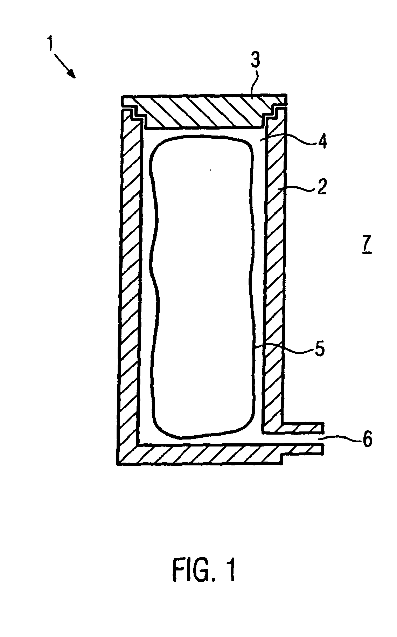

In Fig. 1 ist eine hydrostatische Presse 1 gezeigt, in die ein gummielastischer Körper 5 eingesetzt ist, der seinerseits mit einem Pulver wie beispielsweise einem Zirkonoxidpulver und Zusätzen gefüllt ist.

Der gummielastische Körper 5 befindet sich in einem Innenraum 4, der durch einen Behälter 2 und einen Deckel 3 geschaffen wird. Durch eine Zuleitung 6 kann hydrostatischer Druck auf dem gummielastischen Körper 5 und somit auf das im gummielastischen Körper 5 befindlichen Pulver ausgeübt werden.

Der gummielastische Körper 5 wurde in einem Sauberraum 7 mit dem Pulver gefüllt und verschlossen.

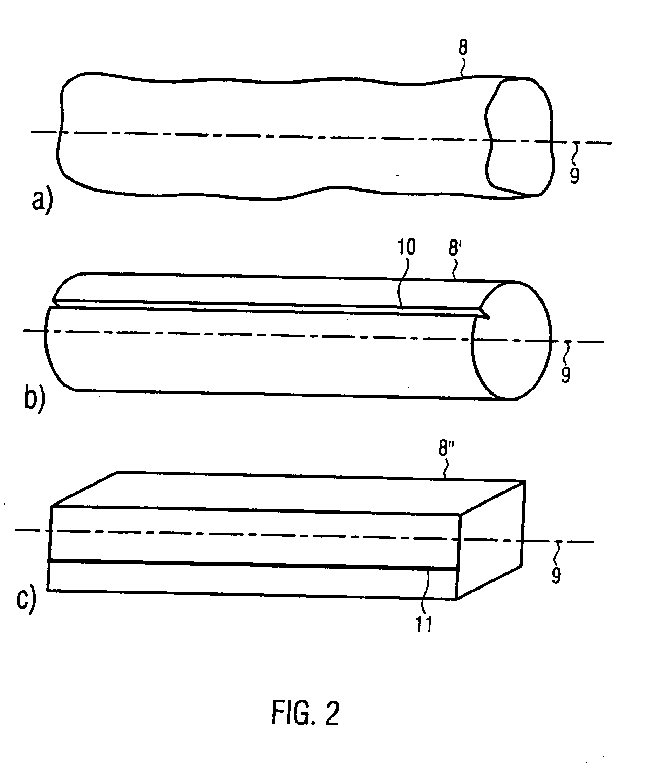

Ein mit der hydrostatischen Presse aus Fig. 1 gewonnener Rohlingskörper ist in Fig. 2a schematisch dargestellt. Der Rohlingskörper 8 weist eine Längsachse 9 auf sowie weiterhin eine etwas unregelmäßig gewellte Oberfläche. Durch Überdrehen des Rohlings 8 kann dieser in einen rotationssymmetrischen Rohlingskörper 8' (siehe Fig. 2b) umgearbeitet werden. In diesem Rohlingskörper 8' kann auch eine Nut 10 vorgesehen werden, mit der Rohlinge in ihrer Lage nach dem Zerteilen des Rohlingskörpers 8' zugeordnet oder erkannt werden. Hierfür ist/sind insbesondere eine Nut mit einem asymmetrischen Querschnitt oder mehrere asymmetrisch angeordnete Nuten vorteilhaft.

Die hydrostatische Presse arbeitet bei Umgebungstemperatur. Es sind aber auch erhöhte Temperaturen von bis zu 50 °C, 100 °C oder 200 °C möglich.

In Fig. 2c ist eine bevorzugte Ausführungsform eines solchen Rohlingskörpers 8" gezeigt, der plan bearbeitet wurde, so dass sich ein quadratischer oder rechteckiger Querschnitt ergibt. Der Rohlingskörper wird mit einer Nut oder Markierung 11 versehen, die vorzugsweise derart angeordnet ist, dass sie nicht auf einer Symmetrie-Ebene oder Achse des Rohlingskörpers 2a liegt, so dass eine eindeutige Lageerkennung des Rohlings, der aus diesem Rohlingskörper 8" gewonnen wird, möglich ist. Auch hier sind asymmetrisch und/oder mehrere asymmetrisch angeordnete Nuten vorteilhaft.

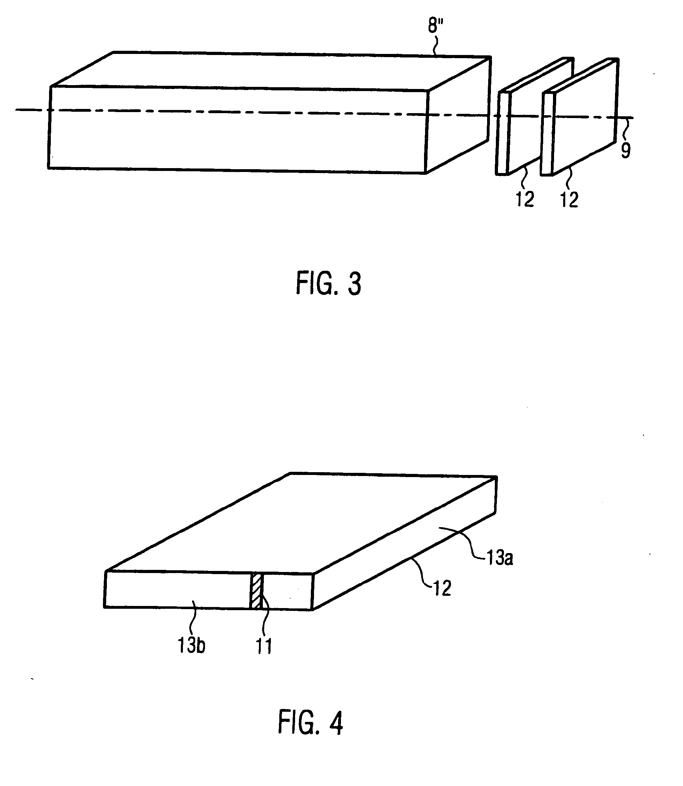

In Fig. 3 ist beispielhaft gezeigt, wie der Rohling 8" durch Trennen entlang von Ebenen senkrecht zu der Achse 9 in einzelne Rohlinge 12 zerteilt wird.

Statt erst Rohlingskörper 8 zu gewinnen, die anschießend zerteilt werden, ist es jedoch auch möglich, unmittelbar Rohlinge zu pressen. Das Zertrennen in Rohlinge kann dann entfallen. Die Oberflächen von durch Pressen gewonnenen Rohlingen werden in der Regel auch leicht uneben sein, so dass sich hier eine Planbearbeitung dieser Oberflächen anbietet, um Rohlinge mit einfachen geometrischen Formen zu erhalten.

In Fig. 4 ist schematisch ein Rohling 12 dargestellt. Dieser Rohling hat eine Scheibenform mit Scheibenseiten 13a und 13b.

Die Seiten 13a und 13b sind stufenlos, d. h. in der Richtung von der oberen zur unteren Seite ist die Seite ohne Stufen. In einer Richtung senkrecht dazu können Stufen z. B. durch eine Nut gegeben sein.

Auf der Seite 13b ist weiterhin eine Markierung zu erkennen, die der Markierung 11 aus der Fig. 2c entspricht. Da diese Markierung nicht mittig auf der Seite 13b angebracht ist (was jedoch auch möglich wäre), kann durch das Finden der Markierung 11 die Lage des Rohlings 12 eindeutig zugeordnet werden. Wird der Rohling beispielsweise um 180° gedreht und zwar um eine Achse, die in der Scheibenebene liegt und durch die Fläche 13b hindurchgeht, so befindet sich die Markierung 11 weiter links statt, wie die Fig. 4, weiter rechts von der Mitte. Weiterhin wird durch die Markierung 11 eine der vier Scheibenseiten ausgezeichnet, so dass sich mit der Markierung 11 die Lage des Rohlings 12 eindeutig bestimmen lässt. -

Ein solcher Rohling, wie er in Fig. 4 dargestellt ist, weist eine 3-Punkt-Biegefestigkeit zwischen 20 und 180 MPa auf. Nach dem Dichtsintern eines solchen Rohlings- bzw. von einem aus diesem Rohling gewonnenen Zahnersatzteil beispielsweise durch Fräsen, ergibt sich ein Material mit einer 3-Punkt-Biegefestigkeit von mindestens 1300 MPa.

Hier sind auch Werte von bis zu über 1500 oder auch höher als 1600 und 1700, 1800 oder sogar über 1900 MPa möglich.

Diese Werte sind auch erreichbar wenn das Dichtsintern bei Umgebungsdruck durchgeführt wird. Leicht erhöhter Druck von bis zu 1, 2, 5, 10, 20, 50, 100 oder 200 bar ist jedoch auch möglich.

## Patentansprüche

1. Verfahren zum Herstellen von einem Zahnersatzteil oder von Zahnersatzteilen mit den Schritten:
a) - Herstellen von Rohlingen (12) oder Rohlingskörpern (8) durch Pressen eines keramischen Materials in Pulverform mit einem isostatischen oder uniaxialen, biaxial, oder triaxialen Pressdruck von mindestens 1500 bar und nicht mehr als 4100 bar, wobei 1 bar dem Wert von 10⁵ Pa entspricht, zu einem Rohling (12) oder einem Rohlingskörper (8), wobei beim maximalen Pressdruck oder beim gesamten Pressvorgang Raumtemperatur oder eine Temperatur von weniger als 200 °C angelegt ist, wodurch die Rohlingskörper (8) oder die Rohlinge (12) 3-Punkt-Biegefestigkeiten im Bereich von zwischen 20 MPa und 180 MPa aufweisen, und wobei Rohlingskörper (8) in mehrere Rohlinge (12) zerlegt werden, wobei dies vorzugsweise durch Trennen entlang einer Ebene geschieht, die vorzugsweise in etwa senkrecht zu einer Längsachse (9) des Rohlingskörpers (8) liegt;
oder
- Bereitstellen eines Rohlings (12), wobei der Rohling (12) erhaltbar ist durch isostatisches oder uniaxiales, biaxiales oder triaxiales Pressen eines keramischen Materials bei einem Pressdruck von mindestens 1500 bar und nicht mehr als 4100 bar, wobei beim maximalen Pressdruck oder beim gesamten Pressvorgang Raumtemperatur oder eine Temperatur von weniger als 200 °C angelegt ist, wobei der Rohling (12) nach dem Pressen eine 3-Punkt-Biegefestigkeit im Bereich von zwischen 20 MPa und 180 MPa aufweist;
oder
- Bereitstellen von einem Satz von Rohlingen aus ein und demselben Rohlingskörper (8), wobei der Rohlingskörper (8) durch Pressen eines keramischen Materials bei einem isostatischen, uniaxialen, biaxialen oder triaxialen Pressdruck von mindestens 1500 bar und nicht mehr als 4100 bar erhaltbar ist, wobei beim maximalen Pressdruck oder beim gesamten Pressvorgang Raumtemperatur oder eine Temperatur von weniger als 200 °C angelegt ist, wobei der Rohlingskörper (8) nach dem Pressen eine 3-Punkt-Biegefestigkeit im Bereich von zwischen 20 MPa und 180 MPa aufweist;
b) Bearbeiten des Rohlings oder der Rohlinge des Rohlingssatzes, um ein Zahnersatzteil oder mehrere Zahnersatzeile zu formen, beispielsweise durch Fräsen
wobei das Zahnersatzteil oder die Zahnersatzteile nach dem Schritt b) dichtgesintert wird/werden,
wobei dichtgesintert wird, vorzugsweise bei einer Temperatur zwischen 1200 °C und 1700 °C und zwar für eine Zeit von 10 Stunden bis 16 Stunden, bevorzugt 14 Stunden, wobei vorzugsweise das gesamte Dichtsintern oder das Anlegen der Maximaltemperatur bei Umgebungsdruck oder einem Überdruck von weniger als 200 bar durchgeführt wird, wobei das keramische Material nach dem Dichtsintern eine 3-Punkt-Biegefestigkeit von mindestens 1300 MPa bis zu 2000 MPa aufweist.

2. Das Verfahren nach Anspruch 1, wobei der Rohling (12) eine Scheibenform hat, wobei der Rohling (12) vorzugsweise kreisrund, quadratisch, rechteckig oder polygon ist.

3. Das Verfahren nach Anspruch 2, wobei der Rohling (12) an der Scheibenseite (13b) eine Markierung (11) wie etwa eine Nut und/oder eine farbige Linie und/oder einen Barcode aufweist.

4. Das Verfahren nach Anspruch 2 oder 3, wobei die Scheibenseite (13a, 13b) stufenlos ist.

5. Das Verfahren nach einem der Ansprüche 1 bis 4, wobei das Pulver zur Pressung in einen gummielastischen Körper (5) eingefüllt wird.

6. Verfahren nach Anspruch 5, wobei das Befüllen in einem Sauberraum (7) vorgenommen wird.

7. Verfahren nach einem der Ansprüche 1 bis 6, wobei das Pressen mit einer hydrostatischen Presse (1) durchgeführt wird.

8. Verfahren nach einem der Ansprüche 1 bis 7, wobei der Rohlingskörper (8) oder der Rohling (12) nach dem Pressen überdreht oder planbearbeitet wird.

## Claims

1. Method for producing a denture part or denture parts, comprising the steps of:
a) - producing of blanks (12) or blank bodies (8) by pressing of a powdery ceramic material with an isostatic or uniaxial, biaxial or triaxial pressing pressure of at least 1500 bar and not more than 4100 bar, wherein 1 bar corresponds the value of 10⁵ Pa, to a blank (12) or a blank body (8), wherein at the maximum pressing pressure or throughout the whole pressing operation room temperature or a temperature of less than 200°C is applied, whereby the blank bodies (8) or blanks (12) have 3-point bending strengths in the region between 20 MPa and 180 MPa and wherein blank bodies (8) are separated into several blanks (12), wherein this is preferably carried out by separation along a plane which is preferably approximately perpendicular to a longitudinal axis (9) of the blank body (8);
or
- providing a blank (12), wherein the blank (12) is obtainable by isostatic or uniaxial, biaxial or triaxial pressing of a ceramic material at a pressing pressure of at least 1500 bar and not more than 4100 bar, wherein at the maximum pressing pressure or throughout the whole pressing operation room temperature or a temperature of less than 200°C is applied, whereby the blank (12) has a 3-point bending strength in the region between 20 MPa and 180 MPa after pressing;
or
- providing a set of blanks of one and the same blank body (8), wherein the blank body (8) is obtainable by pressing of a ceramic material at an isostatic, uniaxial, biaxial or triaxial pressing pressure of at least 1500 bar and not more than 4100 bar, wherein at the maximum pressing pressure or throughout the whole pressing operation room temperature or a temperature of less than 200°C is applied, wherein the blank body (8) has a 3-point bending strength in the region between 20 MPa and 180MPa after pressing;
b) machining the blank or the blanks of the set of blanks to form a denture part or a plurality of denture parts, e.g. by milling,
wherein the denture part or the denture parts is/are dense sintered after the step b),
wherein dense sintering is performed, preferably at a temperature between 1200°C and 1700°C and namely for a period of 10 hours to 16 hours, preferably 14 hours, wherein
preferably the whole dense sintering operation or the application of the maximum pressure is carried out at ambient pressure or at an overpressure of less than 200 bar, wherein the ceramic material has a 3-point bending strength of at least 1300 MPa up to 2000 MPa after dense sintering.

2. The method according to claim 1, wherein the blank (12) has the shape of a plate, wherein the blank (12) is preferably circular, square, rectangular or polygonal.

3. The method according to claim 2, wherein at the plate side (13b) the blank (12) has a marking (11), such as a groove and/or a colored line and/or a bar code.

4. The method according to claim 2 or 3, wherein the plate side (13a, 13b) is stepless.

5. The method according to any one of claims 1 to 4, wherein the powder is filled into a rubber-elastic body (5) for pressing.

6. Method according to claim 5, wherein the filling operation is carried out in a cleanroom (7).

7. Method according to any one of claims 1 to 6, wherein the pressing operation is carried out with a hydrostatic press (1).

8. Method according to any one of claims 1 to 7, wherein the blank body (8) or the blank (12) is lathe turned or plane machined after pressing.

## Revendications

1. Procédé pour fabriquer un élément ou des éléments de prothèse dentaire, avec les étapes qui consistent à :
a)- fabriquer des ébauches (12) ou des corps d'ébauche (8) par moulage par compression d'un matériau céramique sous forme de poudre avec une pression de moulage isostatique ou uniaxiale, biaxiale ou triaxiale d'au moins 1500 bar et pas plus de 4100 bar, 1 bar correspondant à la valeur de 10⁵ Pa, pour obtenir une ébauche (12) ou un corps d'ébauche (8), étant précisé qu'en présence de la pression de moulage maximale ou lors de la totalité de l'opération de moulage par compression, la température ambiante ou une température de moins de 200°C est appliquée, moyennant quoi les corps d'ébauche (8) ou les ébauches (12) présentent des résistances à la flexion en trois points situées dans la plage entre 20 MPa et 180 MPa, et étant précisé que les corps d'ébauche (8) sont divisés en plusieurs ébauches (12), et ce de préférence par séparation le long d'un plan qui est de préférence à peu près perpendiculaire à une axe longitudinal (9) du corps d'ébauche (8) ;
ou
- préparer une ébauche (12), étant précisé que l'ébauche (12) peut être obtenue par moulage par compression isostatique ou uniaxial, biaxial ou triaxial d'un matériau céramique avec une pression de moulage d'au moins 1500 bar et pas plus de 4100 bar, étant précisé qu'en présence de la pression de moulage maximale ou lors de la totalité de l'opération de moulage par compression, la température ambiante ou une température de moins de 200°C est appliquée, moyennant quoi l'ébauche (12) présente après le moulage par compression une résistance à la flexion en trois points située dans la plage entre 20 MPa et 180 MPa ;
ou
- préparer une série d'ébauches à partir d'un même corps d'ébauche (8), étant précisé que le corps d'ébauche (8) peut être obtenu par moulage par compression d'un matériau céramique à une pression de moulage isostatique, uniaxiale, biaxiale ou triaxiale d'au moins 1500 bar et pas plus de 4100 bar, et qu'en présence de la pression de moulage maximale ou lors de la totalité de l'opération de moulage par compression, la température ambiante ou une température de moins de 200°C est appliquée, moyennant quoi le corps d'ébauche (8) présente après le moulage par compression une résistance à la flexion en trois points située dans la plage entre 20 MPa et 180 Mpa ;
b)- traiter l'ébauche ou les ébauches de ladite série afin de former un ou plusieurs éléments de prothèse dentaire, par exemple par fraisage,
étant précisé que l'élément de prothèse dentaire ou les éléments de prothèse dentaire sont soumis à un frittage à densité maximale après l'étape b),
que le frittage à densité maximale est réalisé de préférence à une température située entre 1200°C et 1700°C, pendant 10 à 16 heures, de préférence 14 heures, que la totalité du frittage à densité maximale ou l'application de la température maximale sont réalisés à la pression ambiante ou à une surpression de moins de 200 bar, et que le matériau céramique présente après le frittage à densité maximale une résistance au pliage en 3 points d'au moins 1300 MPa à 2000 MPa.

2. Procédé selon la revendication 1, étant précisé que l'ébauche (12) a une forme d'une plaque, l'ébauche (12) étant de préférence ronde, carrée, rectangulaire ou polygonale.

3. Procédé selon la revendication 2, étant précisé que l'ébauche (12) présente sur le côté de plaque (13b) un marquage (11) tel qu'une rainure et/ou une ligne de couleur et/ou un code-barres.

4. Procédé selon la revendication 2 ou 3, étant précisé que le côté de plaque (13a, 13b) est non étagé.

5. Procédé selon l'une des revendications 1 à 4, étant précisé que la poudre est versée, en vue du moulage par compression, dans un corps élastique (5).

6. Procédé selon la revendication 5, étant précisé que le remplissage est réalisé dans une chambre blanche (7).

7. Procédé selon l'une des revendications 1 à 6, étant précisé que le moulage par compression est réalisé avec une presse hydrostatique (1).

8. Procédé selon l'une des revendications 1 à 7, étant précisé que le corps d'ébauche (8) ou l'ébauche (12) sont traités par tournage ou dressage, après le moulage par compression.
